# EUROPEAN PATENT SPECIFICATION

(11) **EP 1 145 001 B1**
(45) Date of publication and mention of the grant of the patent: **10.05.2006**
(21) Application number: 00900790.7
(22) Date of filing: 17.01.2000
(51) Int. Cl.: G01N 33/50, C12Q 1/02, A61K 35/74

(54) **(IN VITRO) MODEL FOR GASTROINTESTINAL INFLAMMATION**
IN VITRO MODELL FÜR GASTROINTESTINALE ENTZÜNDUNGEN
MODELE (IN VITRO) RELATIF A UNE INFLAMMATION GASTRO-INTESTINALE

(30) Priority: 15.01.1999 IE 990033; 20.09.1999 IE 990782
(43) Date of publication of application: 17.10.2001
(73) Proprietor: Enterprise Ireland (trading as BioResearch Ireland), Dublin 9 (IE); University College Cork - National University of Ireland, Cork, Cork (IE)
(72) Inventor: COLLINS, John, Kevin, Doughcloyne, County Cork (IE); O'MAHONY, Liam, Cork (IE)
(74) Representative: O'Brien, John Augustine
(86) International application number: PCT/IE2000/000009
(87) International publication number: WO 2000/042429

(56) References cited:
- SOTO A ET AL: "Cytokine release and mitogenic activity in the viridans streptococcal shock syndrome." CYTOKINE, vol. 10, no. 5, May 1998 (1998-05), pages 370-376, XP000929269 ISSN: 1043-4666
- ARNOLD R ET AL: "Cytokine (IL-8, IL-6, TNF-alpha) and soluble TNF receptor-I release from human peripheral blood mononuclear cells after respiratory syncytial virus infection." IMMUNOLOGY, vol. 85, no. 3, 1995, pages 364-372, XP002143230 ISSN: 0019-2805
- MCKAY D M ET AL: "T cell-monocyte interactions regulate epithelial physiology in a coculture model of inflammation." AMERICAN JOURNAL OF PHYSIOLOGY, (1996). 270 (2 PT. 1):C418-28. ISSN: 0002-9513., XP000929273
- ATCC QUARTERLY NEWSLETTER, vol. 13, no. 1, 1993, pages 1-1-11,
- TRENDS BIOTECHNOL, vol. 12, no. 1, January 1994 (1994-01), pages 6-8,

## Description

### Introduction

The invention relates to a system for studying intercellular communication and in particular to an *in vitro* model for screening for promoters and inhibitors of inflammation.

The problem of providing suitable model systems to mimic *in vivo* normal and disease states is well known.

An improved tissue culture apparatus, the Transwell™ has been developed and is described in US5,026,649. In this system cells or tissue samples are separated from a nutrient medium by a permeable membrane. A concentration gradient of nutrients may then develop and feed the cells through the permeable membrane more closely reflecting the situation *in vivo*. The Transwell™ has been used to mimic various *in vivo* normal and disease states.

Gastrointestinal inflammation has become one of the major health concerns in the western world. There is therefore a need to provide an *in vitro* model to mimic the *in vivo* inflammation state for screening for promoters and inhibitors of inflammation.

Inflammation is the term used to describe the local accumulation of fluid, plasma proteins and white blood cells at a site that has sustained physical damage, infection or where there is an ongoing immune response. Inflammation is traditionally defined by the Latin words, dolor, rubor, calor and tumor meaning pain, redness, heat and swelling. Dilation and increased permeability of local blood vessels, due to vasoactive plasma proteins, reactive oxygen intermediates, nitrous oxide, certain products of arachidonic acid metabolism, histamine and serotonin, allows for increased blood flow and vascular permeability leading to the redness, heat and swelling. Endothelial adhesive properties also change causing circulating white blood cells to stick allowing migration into the inflammatory site. The release of mediators, such as bradykinin, within the inflammatory site causes the pain. The first cells to arrive at an inflammatory site include the phagocytic neutrophils followed by monocytes and later by lymphocytes that have been activated in lymph nodes draining the site. During an inflammatory state certain systemic effects such as fever and acute phase protein production can also be observed. Following eradication of the offending organism and repair of the damaged tissue, the inflammation normally subsides.

Control of the inflammatory response is exerted on a number of levels. The controlling factors include hormones (e.g. hydrocortisone), prostaglandins, reactive oxygen and nitrogen intermediates, leukotrienes and cytokines. Cytokines are low molecular weight biologically active proteins that are involved in the generation and control of immunological and inflammatory responses, while also regulating development, tissue repair and haematopoiesis. They provide a means of communication between leukocytes themselves and also with other cell types. A number of cell types produce these cytokines. Neutrophils, monocytes and lymphocytes are the major sources of these cytokines during inflammatory reactions due to their large numbers at the injured site. Other resident cell types may also produce cytokines. For example, injury to the skin results in keratinocyte release of IL-1 (Kupper, T.S. J Clin Invest 1990 Dec;86(6):1783-9). Platelets contain stores of TGFβ, PDGF and EGF and damage to endothelial cells results in platelet aggregation and release of stored cytokine.

Multiple mechanisms exist by which cytokines generated at inflammatory sites influence the inflammatory response. Chemotaxis stimulates homing of inflammatory cells to the injured site. A number of cytokines are chemotactic factors, such as TGFβ (Brandes ME, et al. J Immunol 1991 Sep 1;147(5):1600-6) and IL-8 (Baggiolini M, et al. Int J Immunopharmacol 1995 Feb;17(2):103-8). Factors other than cytokines are also chemotactic, such as the complement component C5a and leukotriene B₄. Cytokines promote infiltration of cells into tissue by increasing expression of adhesion molecules on endothelial cells and leukocytes (Springer TA. Scand J Immunol 1990 Sep;32(3):211-6). Cytokines released within the injured tissue result in activation of the inflammatory infiltrate. Stimulation of the release of reactive oxygen intermediates, protease containing granules and additional cytokines from monocytes and neutrophils maintain the inflammatory state. Most cytokines are pleiotrophic and express multiple biologically overlapping activities. Cytokine cascades and networks control the inflammatory response rather than the action of a particular cytokine on a particular cell type (Arai KI, et al. Annu Rev Biochem 1990;59:783-836). However, TNFα and IL-1β have been demonstrated to be the pivotal cytokine mediators in stimulating inflammatory responses. Waning of the inflammatory response results in lower concentrations of the appropriate activating signals and other inflammatory mediators leading to the cessation of the inflammatory response. However, the factors that control the resolution of inflammatory lesions are very poorly understood.

The inflammatory response is critical to the defence and repair of host tissues. However, uncontrolled responses can result in significant tissue and organ damage and may result in the death of the host (Dinarello CA, et al., Rev Infect Dis 1988 Jan-Feb;10(1):168-89). Examples of such disease states include inflammatory bowel disease, rheumatoid arthritis, sepsis, psoriasis, contact dermatitis, multiple sclerosis, atherosclerosis, sarcoidosis, idiopathic pulmonary fibrosis, dermatomyositis, hepatitis, diabetes, allograft rejection, graft versus host disease and certain para-neoplastic phenomena. Thus, inhibition of these destructive inflammatory responses is a compelling therapeutic option.

As many cytokines may have both pro and anti-inflammatory activities, patterns or networks of cytokine release have been associated with different types of immune responses. The existence of T cells which differ in their pattern of cytokine secretion allows differentiation of inflammatory or immune responses into at least two categories, cell mediated or humoral responses (for review see Abbas AK, et al., Nature 1996 Oct 31;383(6603):787-93). Th1 responses are categorised by IFNγ, TNFβ and IL-2 production leading to a cell-mediated response while Th2 cells secrete IL-4, IL-5, IL-9, IL-10 and IL-13 resulting in a humoral response (Mosmann TR, et al., J Immunol 1986 Apr 1;136(7):2348-57). Differentiation of T cells into either network depends on the cytokine milieu in which the original antigen priming occurs (Seder RA, et al., J Exp Med 1992 Oct 1;176(4):1091-8). These primary immune responses may be influenced by a number of cell types including γδ T cells (Ferrick DA, et al., Nature 1995 Jan 19;373(6511):255-7) and also mast cells, which at mucosal sites seem to contain IL-4, IL-10 and IL-13 which are anti-inflammatory and Th2 type cytokines (Marietta EV, et al., Eur J Immunol 1996 Jan;26(1):49-56). Different types of stimulation may also direct this response such as immune complex deposition within inflammatory sites, which increase IL-6 and IL-10 production and inhibits production of TNFα and IL-1β thus influencing the Th1/Th2 balance (Berger S, et al., Eur J Immunol 1996 Jun;26(6): 1297-301.). For successful elimination of some pathogens, the correct cytokine network needs to be established, such as the intracellular bacterium *Listeria monocytogenes* which elicits a Th1 response while the extracellular parasite *Nippostrongylus brasiliensis* requires a Th2 response (Ferrick et al., 1995). Each of these T cell subsets produce cytokines that are autocrine growth factors for that subset and promote differentiation of naive T cells into that subset (for review see Trinchieri G, et al., Cytokine Growth Factor Rev 1996 Aug;7(2):123-32). These two subsets also produce cytokines that cross-regulate each other's development and activity. IFNγ amplifies Th1 development and inhibits proliferation of Th2 T cells (Fitch FW, et al., Annu Rev Immunol 1993;11:29-48) while IL-10 blocks Th1 activation.

The production of these multifunctional cytokines across a wide spectrum of tumour types suggests that significant inflammatory responses are ongoing in patients with cancer. It is currently unclear what protective effect this response has against the growth and development of tumour cells *in vivo.* However, these inflammatory responses could adversely affect the tumour bearing host. Complex cytokine interactions are involved in the regulation of cytokine production and cell proliferation within tumour and normal tissues (McGee DW, et al., Immunology 1995 Sep;86(1):6-11..,1995, Wu S, et al., Gynecol Oncol 1994 Apr:53(1):59-63). It has long been recognised that weight loss (Inagaki J, et al., Cancer 1974 Feb:33(2):568-73.) and initial malnutrition indicates a poor prognosis (Van Eys J. Nutr Rev 1982 Dec: 40(12):353-9). For a tumour to grow and spread it must induce the formation of new blood vessels and degrade the extracellular matrix. The inflammatory response may have significant roles to play in the above mechanisms, thus contributing to the decline of the host and progression of the tumour.

Thus, the initiation of inflammatory responses, and the subsequent tissue damage, is dependent on environmental factors such as the inflammatory agent itself, the specific cell types present and the cytokine milieu initially present. *In vitro* systems must therefore incorporate all these conditions in order to reliably assess the inflammatory potential of novel compounds. Currently these *in vitro* systems use isolated populations of immune cells, such as peripheral blood mononuclear cells (PBMCs).

A culture system showing the stimulation of inflammatory mediators by a virus has been described (Arnold et al, Immunology Vol. 85, No. 3, 1995, pages 364-372). The system comprises human PBMCs and a monolayer of pulmonary epithelial A549 cells co-cultured and then exposed to respiratory syncytial virus.

The present invention is directed to a cell culture system to serve as a model for gastrointestinal inflammation and a method for investigating intercellular communication and for screening for promoters and inhibitors of inflammation as defined by the claims.

### Statements of Invention

According to the invention there is provided an in vitro method for testing the inflammatory effect of a material including probiotic microorganisms comprising introducing material including probiotic microorganisms comprising or suspected of comprising an inflammatory agent into a system comprising epithelial cells of gastrointestinal, respiratory or genitourinary origin which interact with the immune system and peripheral blood mononuclear cells and determining the change in an immunological marker in response to the material.

Preferably the cells which interact with the immune system and the peripheral blood mononuclear cells are of matched origin.

In another embodiment the peripheral blood mononuclear cells are of gastrointestinal, respiratory or genitourinary origin.

Preferably the cells which interact with the immune system are in the form of a monolayer. Most preferably the cells are on a microporous support.

In one embodiment the invention provides a method for testing the inflammatory effect of a material including probiotic microorganisms comprising the steps of: -
placing a microporous support having a layer of epithelial cells of gastrointestinal, respiratory or genitourinary origin thereon in contact with a nutrient medium in a culture well;
introducing a composition containing peripheral blood mononuclear cells to the medium;
subsequently introducing a material including a probiotic material comprising or suspected of comprising an inflammatory agent to either one or both cells; and
determining the change in immunological marker in response to the material.

Ideally the immunological marker is a cytokine such as TNFα or IL8.

In one embodiment the invention provides a method wherein the immunomodulatory effect is an anti-inflammatory effect.

In another embodiment the invention provides a method wherein the immunomodulatory effect is a pro-inflammatory effect.

Most preferably the test material including probiotic microorganisms includes a strain of *Bifidobacterium*. Strains of this type are referred to in WO 00/42168.

One strain of *Bifidobacterium* is *Bifidobacterium longum infantis* UCC 35624 or a mutant or strain thereof. A deposit of *Bifidobacterium longum infantis* UCC 35624 was made at the National Collections of Industrial and Marine Bacteria Limited (NCIMB) on January 13, 1999 and accorded the accession number NCIMB 41003.

Preferably the test material including probiotic microorganisms includes a strain of *lactobacillus*. Strains of *Lactobacillus salivarius* are disclosed in WO 98/35014.

One strain of *Lactobacillus salivarius* is *Lactobacillus salivarius* strain UCC 118 or a mutant or variant thereof. A deposit of *Lactobacillus salivarius* strain UCC 118 was made at the NCIMB on November 27, 1996 and accorded the accession number NCIMB 40829.

### Brief Description of the Drawings

The invention will be more clearly understood from the following description given by way of example only with reference to the accompanying drawings in which:-
Fig. 1 is a schematic representation of a culture system according to the invention;
Fig. 2 is a schematic representation of a cell signalling in the test system of the invention;
Fig. 3 is a bar chart of TNFα levels in various culture cell systems;
Fig. 4 is a bar chart of TNFα levels in the presence of various bacterial strains;
Fig. 5 is a bar chart of TNFα levels in patient and control samples in the presence of peripheral blood mononuclear cells (PBMCs) and *Bifidobacterium longum infantis* UCC 35624;
Fig. 6 is a bar chart of TNFα and IL-8 levels in the presence of PBMCs, *Bifidobacterium longum infantis* UCC 35624 and epithelial cells; and
Fig. 7 is a graph of extracellular TNFα, IL-1RA, IL-6, sIL-6R, and IFNα levels following exposure to *Lactobacillus salivarius* UCC118.

### Detailed Description

We have developed an *in vitro* cell culture model of gastrointestinal inflammation that provides a valuable means of examining the *in vivo* cellular activity during inflammation. The system includes gastrointestinal epithelial cells as these cells release cytokines that may have powerful influences on local immune response. The model has been used to investigate intercellular communication and to screen for inflammatory agents. The model has also been used to test the role of probiotic bacteria in the modulation of inflammatory cytokines.

The culture model of the present invention may also be utilised for the identification of novel inhibitors and promoters of cytokine production. Compounds have been shown by this model to inhibit or promote the inflammatory response and thus have therapeutic value. Detecting their ability to modulate cytokine production identifies inhibitors or promoters. The assay of this invention also has diagnostic utilities for detecting promoters of inflammatory responses from within inflammatory lesions.

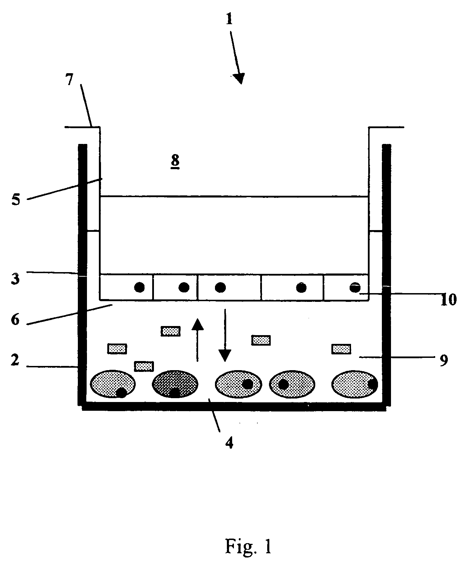

Referring to Fig. 1 there is illustrated a cell culture system 1 according to the invention. A single culture well 2 of a tissue culture cluster plate, typically having 6, 12 or 24 wells, is shown. Such a system is commercially available from Costar and is described in US-A-5139951 and US-A-5272083. The well 2 comprises side walls 3 and a base 4. Detachably suspended in the well 2 is a support 5 having as a base a microporous filter 6. The support 5 hangs by a flange 7 at its upper end from the top of the well 2. The flange 7 of the support 5 positions the microporous filter 6 substantially centrally in the well 2. Openings in the side wall of the support 5 provide access for a pipette to add and withdraw fluid from the well.

The filter 6 when in position in the cell culture well 2, divides the well 2 into two compartments, an upper 8 and a lower compartment 9. The microporous filter 6 with a pore size of about 3 microns is preferably of polycarbonate but other polymers that support epithelial cell attachment may be used. The filter acts as a dividing barrier that permits the passage of soluble mediators between the two compartments but does not allow cell-cell contact.

In use, a confluent epithelial cell monolayer 10 is cultured on the upper surface of the microporous filter 6 for 2-8 weeks under standard growth conditions. Epithelial cells suitable for use can be obtained from any *in vivo* source, including but not limited to epithelial cells derived from the human gastrointestinal tract. CaCo-2 cells, or derivatives thereof, are most preferred, and are available commercially from culture collection banks such as the ATCC and ECACC. Procedures for harvesting and culturing epithelial cells are know in the art.

A composition containing cells of the immune system is added to media in the culture well 2 which is in contact with the lower surface of the microporous filter. PBMCs are added to the lower compartment 9.

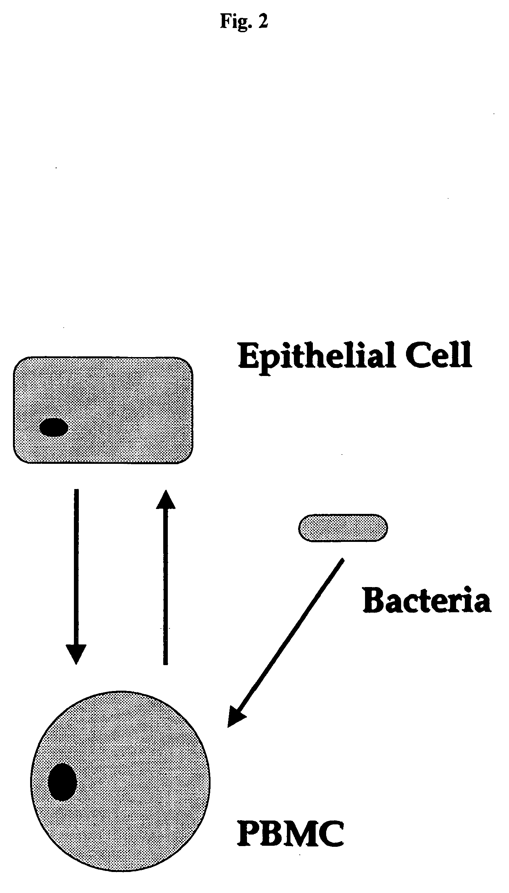

Following shortly after the addition of PBMCs, a composition comprising or suspected of comprising an immunomodulatory agent is added to the culture well. These "test molecules" can be bacterial cells, but are not limited to bacterial components. Whole bacteria and components thereof can be utilised. On addition of the test material a tricellular signalling network is established. This network is schematically illustrated in Figure 2.

The cell culture system and signalling network described above is used in the following examples.

Example 1 Intercellular signalling assay

Epithelial CaCo-2 cells are maintained in 25 cm² culture flasks and upon reaching 80% confluence, the cells are enzymatically (e.g. trypsin) detached from the culture flask and washed in serum free medium. The cells are counted using a haemacytometer, reconstituted in culture medium and added into the cell culture support. The cells attach themselves to the microporous filter and grow outwards forming a monolayer. Epithelial cell monolayers that have reached confluence for a period of time are preferred. These cells are refed every three days.

Peripheral blood mononuclear cells (PBMCs) harvested from peripheral blood by density gradient centrifugation and resuspended in culture medium are added to the culture well. The epithelial cells and PBMCs are separated from each other by the microporous filter which allowed the passage of soluble mediators between the two compartments but did not allow cell-cell contact.

Following shortly after the addition of PBMCs, a composition comprising bacteria which have been previously cultured in broth and washed in antibiotic containing medium is added to the same compartment. The bacteria, PBMCs and epithelial cells are co-incubated for 72 hours at 37°C in a 5% CO₂ atmosphere. Supernatants are then collected and assayed for cytokine concentration using commercially available ELISAs.

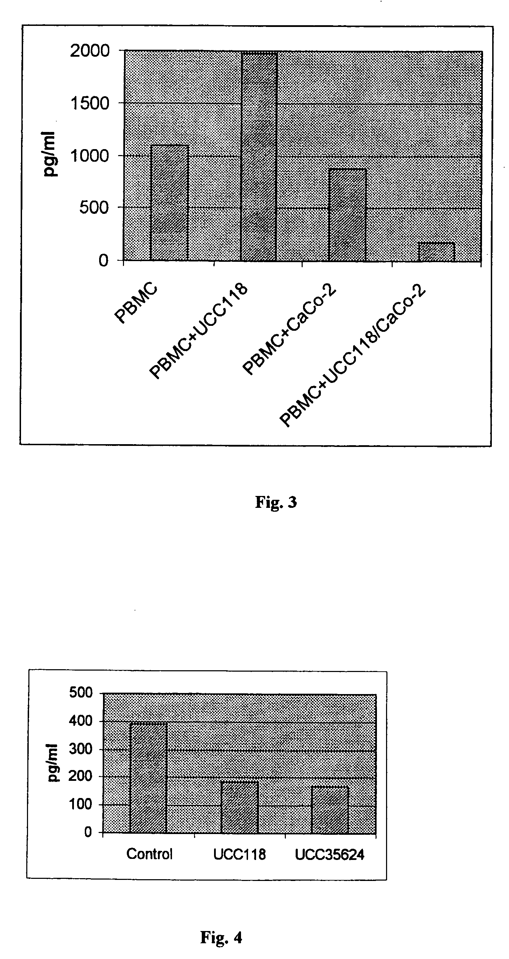

Due to its role as a proinflammatory cytokine, TNFα levels were measured using a Boehringer Mannheim ELISA kit (Catologue no. 1425943). Co-incubation of PBMCs alone with bacteria resulted in the stimulation of TNFα production. Co-incubation of PBMCs, epithelial cells and bacteria resulted in a significant suppression of TNFα production (Figure 3). The results with the tricellular assay are in stark contrast to assays with only two cell types present. It is apparent that the presence of epithelial cells has an immunomodulatory effect on PBMC signalling.

Example 2 Test for anti-inflammatory bacterial strains

A number of lactic acid bacteria, which have been isolated from the human gastrointestinal tract, were examined in this novel assay system as inhibitors of inflammatory responses. All bacterial strains were taken from -20°C glycerol stocks and incubated anaerobically overnight in MRS broth and washed in antibiotic containing medium. Epithelial cell monolayers were grown for 6 weeks prior to the addition of PBMCs and bacterial cells.

The results of these stimulations can be observed in Figure 4. Relative to control cultures, two bacterial strains suppressed TNFα production. The two strains *Lactobacillus salivarius* strain UCC118, which suppressed production of TNFα, is the subject of WO-A-9835014 and a PCT Application filed concurrently with the present application. The *Bifidobacterium longum infantis* strain UCC 35624 is the subject of a PCT Application filed concurrently with the present application.

### Example 3: In vitro studies to examine the immune perception of Bifidobacterium longum infantis.

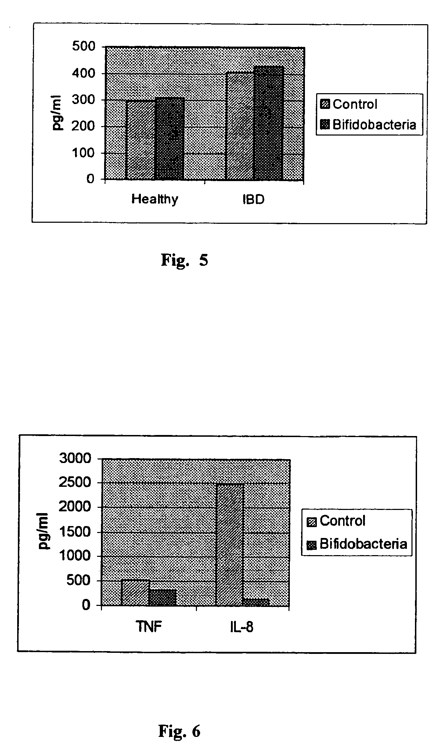

Overnight washed cultures of *Bifidobacteria longum infantis* UCC 35624 were incubated with human peripheral blood mononuclear cells (PBMCs) from both healthy volunteers (n=9) and patients suffering from inflammatory bowel disease (n=5). Production of the proinflammatory cytokine tumour necrosis factor α (TNFα) was measured by ELISA in seventy two hour culture supernatants. Co-incubation of *Bifidobacterium longum infantis* UCC 35624 with human PBMCs did not result in the stimulation of TNFα production (Figure 5). Thus, exposure of the systemic immune system to this bacterium does not induce an inflammatory response.

In order to assess the immune perception of *Bifidobacterium longum infantis* UCC 35624 at mucosal surfaces, co-culturing of epithelial cells and PBMCs was performed in transwell chambers. An epithelial cell monolayer was grown in the upper chamber and PBMCs were incubated in the lower compartment. These were separated from each other by a porous membrane which allowed the passage of soluble mediators between the two compartments but did not allow cell-cell contact. Using this model, the production of TNFα and Interleukin-8 (IL-8) was measured in the presence and absence of *Bifidobacterium longum infantis* UCC 35624 in the PBMC compartment. Co-culture of epithelial cells, PBMCs and *Bifidobacterium longum infantis* resulted in significant suppression of TNFα and IL-8 production (Figure 6). Thus, a tri-cellular network involving epithelial cells, PBMCs and *Bifidobacterium longum infantis* UCC 35624 results in suppression of proinflammatory cytokine production.

### Example 4: In vitro demonstration of the mechanisms underlying the anti-inflammatory effects of Lactobacillus salivarius especially subspecies Salivarius UCC118.

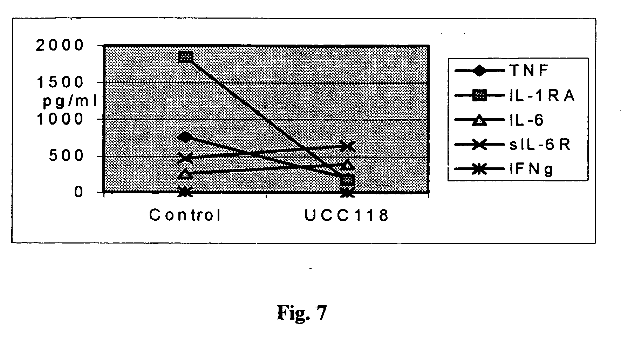

Using the cell culture assay system as described in examples 1 and 2, with epithelial cells as described above and peripheral blood mononuclear cells in the one compartment, extracellular cytokine levels were measured by ELISAs. Following co-incubation with *Lactobacillus salivarius* UCC118, the amount of TNFα produced was significantly reduced compared to control cultures. Furthermore, IL-1RA and IFNγ levels dropped while IL-6 and soluble IL-6 receptor levels increased (Figure 7). Intracellular staining for TNFα confirmed the ELISA result as TNFα levels were lower in the UCC118 stimulated sample compared to controls.

*In vitro* models have demonstrated that *Lactobacillus salivarius* UCC118 is capable of inducing Th2 type cytokines (i.e. interleukin 6 and interleukin 6 soluble receptor) while suppressing the production of inflammatory cytokines such as tumour necrosis factor α and interleukin 1 β. Thus, these results suggest that consumption of *Lactobacillus salivarius* UCC118 would be of benefit to patients suffering from inflammatory diseases, such as IBD.

It will be appreciated that while the specific test models referred to above are used for testing the immunomodulatory especially pro or anti-inflammatory effects of specific probiotic materials the model may also be applied to test any probiotic material including probiotic microorganisms.

It will also be appreciated that white the invention has been described with reference to its application to a specific assay system it may be applied using any suitable assay system as defined by the claims.

## Claims

1. An *in vitro* method for testing the inflammatory effect of a material including probiotic microorganisms comprising introducing a material including probiotic microorganisms comprising or suspected of comprising an inflammatory agent into a system comprising epithelial cells of gastrointestinal, respiratory or genitourinary origin which interact with the immune system, and peripheral blood mononuclear cells and determining the change in an immunological marker in response to the material.

2. A method as claimed in claim 1 wherein the material including probiotic microorganisms includes a strain of *Bifidobacterium*.

3. A method as claimed in claim 1 wherein the material including probiotic microorganisms includes a strain of *Lactobacillus.*

4. A method as claimed in any of claims 1 to 3 wherein the cells which interact with the immune system and the peripheral blood mononuclear cells are of matched origin.

5. A method as claimed in claim 4 wherein the peripheral blood mononuclear cells are of gastrointestinal, respiratory or genitourinary origin.

6. A method as claimed in any preceding claim wherein the cells which interact with the immune system are in the form of a monolayer.

7. A method as claimed in any preceding claim wherein the cells which interact with the immune system are on a microporous support.

8. A method as claimed in claim 1 comprising the steps of: -
placing a microporous support having a layer of epithelial cells of gastrointestinal, respiratory or genitourinary origin thereon in contact with a nutrient medium in a culture well;
introducing a composition containing peripheral blood mononuclear cells to the medium;
subsequently introducing a material including probiotic microorganisms comprising or suspected of comprising an inflammatory agent to either one or both cells; and
determining the change in an immunological marker in response to the material.

9. A method as claimed in any preceding claim wherein the immunological marker is a cytokine.

10. A method as claimed in claim 9 wherein the cytokine is TNFα.

11. A method as claimed in claim 9 wherein the cytokine is IL-8.

12. A method as claimed in any preceding claim wherein the inflammatory effect is an anti-inflammatory effect.

13. A method as claimed in any preceding claim wherein the inflammatory effect is a pro-inflammatory effect.

## Patentansprüche

1. *In-vitro*-Verfahren zur Testung der inflammatorischen Wirkung eines Stoffes, einschließlich probiotischer Mikroorganismen, umfassend die Einführung eines Stoffes, einschließlich probiotischer Mikroorganismen, der ein inflammatorisches Mittel umfasst oder verdächtig ist, dieses zu umfassen, in ein System, das Epithelzellen gastrointestinalen, respiratorischen oder urogenitalen Ursprungs, die mit dem Immunsystem interagieren, und periphäre mononukleäre Blutzellen umfasst, und die Bestimmung der Veränderung in einem immunologischen Marker als Antwort auf den Stoff.

2. Verfahren nach Anspruch 1, worin der Stoff, einschließlich probiotische Mikroorganismen, einen Stamm vom *Bifidobacterium* einschließt.

3. Verfahren nach Anspruch 1, worin der Stoff, einschließlich probiotische Mikroorganismen, einen Stamm vom *Lactobacillus* einschließt.

4. Verfahren nach einem der Ansprüche 1 bis 3, worin die Zellen, die mit dem Immunsystem interagieren, und die periphären mononukleären Blutzellen einem passenden Ursprung entstammen.

5. Verfahren nach Anspruch 4, worin die periphären mononukleären Blutzellen einen gastrointestinalen, respiratorischen oder urogenitalen Ursprung haben.

6. Verfahren nach einem der vorstehenden Ansprüche, worin die Zellen, die mit dem Immunsystem interagieren, in einer Form einer Monoschicht vorliegen.

7. Verfahren nach einem der vorstehenden Ansprüche, worin sich die Zellen, die mit dem Immunsystem interagieren, an einem mikroporösem Träger befinden.

8. Verfahren nach Anspruch 1, das die folgenden Schritte umfasst:
In-Kontakt-Bringen eines mikroporösen Trägers, woran sich eine Schicht von Epithelzellen mit gastrointestinalem, respiratorischem oder urogenitalem Ursprung befindet, mit einem Nährmedium in einem Kulturwell;
Einführung einer Zusammensetzung, die periphäre mononukleäre Blutzellen enthält, in ein Medium;
darauffolgende Einführung eines Stoffes, einschließlich probiotischer Mikroorganismen, der ein inflammatorisches Mittel umfasst oder verdächtig ist, dieses zu umfassen, in entweder eine oder beide Zellen; und
Bestimmung der Veränderung in einem immunologischen Marker als Antwort auf den Stoff.

9. Verfahren nach einem der vorstehenden Ansprüche, worin der immunologische Marker ein Cytokin darstellt.

10. Verfahren nach Anspruch 9, worin das Cytokin TNFα darstellt.

11. Verfahren nach Anspruch 9, worin das Cytokin IL-8 darstellt.

12. Verfahren nach einem der vorstehenden Ansprüche, worin die inflammatorische Wirkung eine anti-inflammatorische Wirkung darstellt.

13. Verfahren nach einem der vorstehenden Ansprüche, worin die inflammatorische Wirkung eine pro-inflammatorische Wirkung darstellt.

## Revendications

1. Méthode in vitro pour tester l'effet inflammatoire d'une substance y compris les microorganismes probiotiques comprenant les étapes consistant à introduire une substance y compris les microorganismes probiotiques comprenant ou suspectée de comprendre un agent inflammatoire dans un système comprenant des cellules épithéliales d'origine gastro-intestinale, respiratoire ou génito-urinaire qui interagissent avec le système immunitaire, et des cellules mononucléaires du sang périphérique, et à déterminer le changement dans un marqueur immunologique en réponse à la substance.

2. Méthode selon la revendication 1, dans laquelle la substance y compris les microorganismes probiotiques inclut une souche de *Bifidobacterium.*

3. Méthode selon la revendication 1, dans laquelle la substance y compris les microorganismes probiotiques inclut une souche de *Lactobacillus.*

4. Méthode selon l'une quelconque des revendications 1 à 3, dans laquelle les cellules qui interagissent avec le système immunitaire et les cellules mononucléaires du sang périphérique sont d'origine appariée.

5. Méthode selon la revendication 4, dans laquelle les cellules mononucléaires du sang périphérique sont d'origine gastro-intestinale, respiratoire ou génito-urinaire.

6. Méthode selon l'une quelconque des revendications précédentes, dans laquelle les cellules qui interagissent avec le système immunitaire sont sous la forme d'une monocouche.

7. Méthode selon l'une quelconque des revendications précédentes, dans laquelle les cellules qui interagissent avec le système immunitaire sont sur un support microporeux.

8. Méthode selon la revendication 1 comprenant les étapes consistant à :
placer un support microporeux comportant une couche de cellules épithéliales d'origine gastro-intestinale, respiratoire ou génito-urinaire disposée sur lui en contact avec un milieu nutritif dans un puits de culture ;
introduire une composition contenant des cellules mononucléaires du sang périphérique dans le milieu ;
introduire par la suite une substance y compris les microorganismes probiotiques comprenant ou suspectée de comprendre un agent inflammatoire contre l'une ou l'autre ou les deux cellules ; et
déterminer le changement dans un marqueur immunologique en réponse à la substance.

9. Méthode selon l'une quelconque des revendications précédentes, dans laquelle le marqueur immunologique est une cytokine.

10. Méthode selon la revendication 9, dans laquelle la cytokine est le TNFα.

11. Méthode selon la revendication 9, dans laquelle la cytokine est l'IL-8.

12. Méthode selon l'une quelconque des revendications précédentes, dans laquelle l'effet inflammatoire est un effet anti-inflammatoire.

13. Méthode selon l'une quelconque des revendications précédentes, dans laquelle l'effet inflammatoire est un effet pro-inflammatoire.
